(19) **Europäisches Patentamt**
**European Patent Office**
**Office européen des brevets**

(11) **EP 4 180 092 A1**

(12) **EUROPEAN PATENT APPLICATION**
published in accordance with Art. 153(4) EPC

(43) Date of publication:
**17.05.2023 Bulletin 2023/20**

(21) Application number: **21843060.1**

(22) Date of filing: **08.07.2021**

(51) International Patent Classification (IPC):
*A61Q 1/02* (2006.01)          *A61Q 1/04* (2006.01)
*A61Q 1/10* (2006.01)          *A61Q 17/04* (2006.01)
*A61Q 19/00* (2006.01)          *A61K 8/37* (2006.01)
*A61K 8/73* (2006.01)

(52) Cooperative Patent Classification (CPC):
**A61K 8/37; A61K 8/73; A61Q 1/02; A61Q 1/04;**
**A61Q 1/10; A61Q 17/04; A61Q 19/00**

(86) International application number:
**PCT/JP2021/025809**

(87) International publication number:
**WO 2022/014463 (20.01.2022 Gazette 2022/03)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **13.07.2020 JP 2020120253**

(71) Applicant: **Daicel Corporation**
**Osaka 530-0011 (JP)**

(72) Inventors:
• **KOBAYASHI, Keiko**
**Tokyo 108-8230 (JP)**

• **OMURA, Masaya**
**Tokyo 108-8230 (JP)**
• **HISAZUMI, Takuya**
**Tokyo 108-8230 (JP)**
• **HAYAMIZU, Hidetaka**
**Tokyo 108-8230 (JP)**
• **IIO, Jumpei**
**Tokyo 108-8230 (JP)**

(74) Representative: **Grünecker Patent- und**
**Rechtsanwälte**
**PartG mbB**
**Leopoldstraße 4**
**80802 München (DE)**

(54) **CELLULOSE ACETATE PARTICLES, COSMETIC COMPOSITION, AND METHOD FOR PRODUCING CELLULOSE ACETATE PARTICLES**

(57)    Provided are cellulose acetate particles. The cellulose acetate particles have an average particle size of 80 nm or greater and 100 μm or less, a sphericity of 0.7 or greater and 1.0 or less, and a relative specific surface area of 3.0 or greater and 20 or less. A total degree of acetyl substitution of the cellulose acetate is 0.7 or greater and 3.0 or less.

SU5000 1.0kV 8.0mm x6.00k SE(L)                    5.00μm

FIG. 3

EP 4 180 092 A1

**Description**

Technical Field

The present disclosure relates to cellulose acetate particles, a cosmetic composition using the same, and a method for producing the same.

Background Art

Various polymer particles which correspond to applications have been proposed. Examples include particles contained in cosmetics, and the purpose of such particles contained in cosmetics varies. The purposes for having particles in cosmetics include improving the spread of the cosmetic, changing the tactile sensation, imparting a wrinkle blurring effect, and improving the lubricity of foundation or the like.

Particularly, particles having a high sphericity are excellent in tactile sensation. To provide excellent tactile sensation, particles contained in cosmetics are required to have a narrow particle size distribution and high sphericity. Examples of such particles include particles made of a synthetic polymer, such as polyamide (such as nylon 12), polymethyl methacrylate (PMMA), and polystyrene (PS).

Typically, polyamide powders have been used as a cosmetic base, especially as a cosmetic base for makeup. This is because a polymer such as polyamide has a refractive index similar to that of sweat, and thus applying the polymer to a face makes the entire face shine and gives gloss to the skin.

In the general process of producing a paste cosmetic or a semi-solid cosmetic, when components of the cosmetic are kneaded with oil, using a large amount of oil enables the components to be well kneaded. However, if the oil used in the production process remains in a cosmetic as a product, a person's face wearing the cosmetic looks greasy, and the person wearing the cosmetic also feels sticky and uncomfortable.

JP S62-215638 A (Patent Document 1) discloses that "each particle of a polyamide powder has a shape close to a true sphere, and the surface of those particles has little unevenness and is smooth. Accordingly, it describes that each particle itself has almost no adsorption capacity, and the absorption capacity of the powder as a whole is significantly low". For such an issue, disclosed are "a method including: dissolving, under heating, a polyamide resin in a lower alcohol solvent containing an anhydrous chloride of an alkaline-earth metal; cooling the resulting product" and "a porous polyamide powder including: a total pore volume of 0.25 cc/g or greater; and a specific surface area of 4.0 m$^2$/g or greater, in which the porous polyamide powder is spherical." However, the sphericity of this powder is insufficient, and it cannot be said that the powder is truly spherical.

In addition, there has been a recent trend to use biodegradable resins due to environmental issues, particularly the issue of microplastics in the ocean. For example, JP 6609726 B (Patent Document 2) discloses cellulose acetate particles having high sphericity, and also describes, as a method for producing the cellulose acetate particles, "a method for producing cellulose acetate particles including: mixing cellulose acetate having a total degree of acetyl substitution of 0.7 or greater and 2.9 or less and a plasticizer to prepare cellulose acetate impregnated with the plasticizer; kneading the cellulose acetate impregnated with the plasticizer and a water-soluble polymer at 200°C or higher and 280°C or lower to prepare a dispersion containing the cellulose acetate impregnated with the plasticizer as a dispersoid; and removing the water-soluble polymer from the dispersion". However, porous cellulose acetate particles cannot be prepared by this method, and there is no sufficient oil absorption.

In addition, attempts have also been made to produce porous particles having biodegradability. For example, JP 2015-214690 A (Patent Document 3) discloses aliphatic polyester resin particles excellent in oil absorbability and suitable for applications such as additives for cosmetics and paints, and a method for producing the aliphatic polyester resin particles. Specifically, it discloses that "the method for producing aliphatic polyester resin particles according to the present invention is a method for producing polymer particles including: dissolving and mixing an aliphatic polyester resin (A), a polymer (B) different from the aliphatic polyester resin, and an organic solvent (C) containing a ketoester compound, forming an emulsion in a system which causes phase separation into two phases of a solution phase mainly containing the aliphatic polyester resin (A) and a solution phase mainly containing the polymer (B) different from the aliphatic polyester resin, and then bringing a poor solvent of the aliphatic polyester resin (A) into contact with the emulsion to precipitate the aliphatic polyester resin (A)". However, this production method is a method for producing particles using a so-called drop method (emulsion polymerization), in which the solid content concentration at the time of production is low, volume shrinkage occurs after a target resin forms a particulate shape, and the resulting particle has a low specific gravity to form a space inside the particle and have a shape that is not a true sphere at all. Accordingly, particles having sufficient biodegradability and tactile sensation cannot be obtained.

JP H06-136175 A (Patent Document 4) discloses that "the present invention provides a method for producing spherical cellulose diacetate particles including: preparing A) a cellulose diacetate solution yielded by dissolving cellulose diacetate in a mixed solvent of a chlorinated hydrocarbon and an alcohol, B) an alcohol-based diluent of an alcohol or an ester and an alcohol, and C) an aqueous medium; adding the alcohol-based diluent B) to the cellulose diacetate solution A) to form a mixed solution; suspending the mixed solution in the aqueous medium C) to form droplets; and then removing the chlorinated hydrocarbon in the droplets through evaporation". However, the production method using such a method for producing particles through suspension does not enable the production of particles having sufficient biodegradability and tactile sensation.

JP 4464815 B (Patent Document 5) discloses "a method for producing particles including: melt-kneading a resin component (A) containing a thermoplastic resin, and a water-soluble auxiliary component (B) containing at least an oligosaccharide (B1) or a plasticizing component (B2) composed of a sugar alcohol to form a dispersion having a sea-island structure in which a continuous phase is composed of the auxiliary component (B); and eluting the auxiliary component (B) from the dispersion, in which the particles are composed of the resin component (A)". However, the production method does not enable the production of particles having sufficient biodegradability and tactile sensation.

Citation List

Patent Document

Patent Document 1: JP S62-215638 A
Patent Document 2: JP 6609726 B
Patent Document 3: JP 2015-214690 A
Patent Document 4: JP H06-136175 A
Patent Document 5: JP 4464815 B

Summary of Invention

Technical Problem

As described above, the use of cellulose acetate (i.e., a material having excellent biodegradability) has not enabled the production of particles excellent in tactile sensation and oil absorbability. An object of the present disclosure is to provide particles excellent in biodegradability, tactile sensation, and oil absorbability.

Solution to Problem

A first aspect of the present disclosure relates to cellulose acetate particles, in which the cellulose acetate particles have an average particle size of 80 nm or greater and 100 μm or less, a sphericity of 0.7 or greater and 1.0 or less, and a relative specific surface area of 3.0 or greater and 20 or less; and a total degree of acetyl substitution of the cellulose acetate is 0.7 or greater and 3.0 or less.

The cellulose acetate particles may have a degree of surface smoothness of 10% or greater and 95% or less.

The cellulose acetate particles may have a bulk specific gravity of 0.2 or greater and 0.7 or less.

In the cellulose acetate particles, the oil absorption using linseed oil may be 60 ml or greater per 100 g of the cellulose acetate particles.

In the cellulose acetate particles, the cellulose acetate may have a total degree of acetyl substitution of 1.6 or greater and less than 2.9.

The cellulose acetate particles may have a relative specific surface area of 10 or greater and 20 or less.

The cellulose acetate particles contain a plasticizer, and a content of the plasticizer is 2 parts by weight or greater and 67 parts by weight or less relative to 100 parts by weight of the cellulose acetate.

In the cellulose acetate particles, the plasticizer may contain at least one selected from the group consisting of a citrate-based plasticizer, a glycerin ester-based plasticizer, and a phthalate-based plasticizer.

A second aspect of the present disclosure relates to a cosmetic composition containing the cellulose acetate particles.

A third aspect of the present disclosure relates to a method for producing cellulose acetate particles including: mixing cellulose acetate having a total degree of acetyl substitution of 0.7 or greater and 3.0 or less, a plasticizer, a first thermoplastic polymer, and a second thermoplastic polymer, and forming a mixture of cellulose acetate containing the plasticizer, the first thermoplastic polymer, and the second thermoplastic polymer; melt-kneading the mixture at 200°C or higher and 280°C or lower; and removing the first thermoplastic polymer and the second thermoplastic polymer from the melt-kneaded mixture, in which when SPa represents an SP value of the cellulose acetate, SPb represents an SP value of the first thermoplastic polymer, and SPc represents an SP value of the second thermoplastic polymer, SPa, SPb, and SPc satisfy the following relationship: $0.1 \leq |$ SPc - SPa $| / |$ SPb - SPa $| \leq 0.9$.

In the method for producing cellulose acetate particles, the mixing may be performed by mixing the cellulose acetate, the plasticizer, the first thermoplastic polymer, and the second thermoplastic polymer at 20°C or higher and lower than 200°C, and then melt-kneading the mixture.

In the method for producing cellulose acetate particles, the first thermoplastic polymer may be a water-soluble polymer.

In the method for producing cellulose acetate particles, the second thermoplastic polymer may be a water-soluble polymer.

In the method for producing cellulose acetate particles, the plasticizer may contain at least one selected from the group consisting of a citrate-based plasticizer, a glycerin ester-based plasticizer, an adipate-based plasticizer, and a phthalate-based plasticizer.

In the method for producing cellulose acetate particles, the plasticizer may contain at least one selected from the group consisting of triethyl citrate, acetyl triethyl citrate, acetyl tributyl citrate, triacetin, diacetin, diisononyl adipate, and diethyl phthalate.

In the method for producing cellulose acetate particles, the plasticizer may contain at least one selected from the group consisting of acetyl triethyl citrate, triacetin, diacetin, and diethyl phthalate.

In the method for producing cellulose acetate particles, the plasticizer may contain at least one selected from the group consisting of acetyl triethyl citrate and triacetin.

In the method for producing cellulose acetate particles, the first thermoplastic polymer may contain at least one selected from the group consisting of polyvinyl alcohol and thermoplastic starch.

In the method for producing cellulose acetate particles, the second thermoplastic polymer may be polyethylene glycol.

Advantageous Effects of Invention

The present disclosure can provide particles excellent in biodegradability, tactile sensation, and oil absorbability.

Brief Description of Drawings

FIG. 1 is a scanning electron microscope (SEM) image (magnification: 3000 times) of cellulose acetate particles of Example A-1.

FIG. 2 is an SEM image (magnification: 5000 times) of cellulose acetate particles of Example A-1.

FIG. 3 is an SEM image (magnification: 6000 times) of cellulose acetate particles of Example A-1, and is a view for explaining a method for evaluating a degree of surface smoothness (%).

FIG. 4 is a binarized image of FIG. 3, and is a view for explaining the method for evaluating a degree of surface smoothness (%).

FIG. 5 is an SEM image (magnification: 3000 times) of cellulose acetate particles of Example A-12.

FIG. 6 is an SEM image (magnification: 5000 times) of cellulose acetate particles of Example A-12.

FIG. 7 is an SEM image (magnification: 3000 times) of cellulose acetate particles of Comparative Example A-1.

FIG. 8 is an SEM image (magnification: 5000 times) of cellulose acetate particles of Comparative Example A-1.

Description of Embodiments

Cellulose Acetate Particles

The cellulose acetate particles of the present disclosure are cellulose acetate particles, in which the cellulose acetate particles have an average particle size of 80 nm or greater and 100 μm or less, a sphericity of 0.7 or greater and 1.0 or less, and a relative specific surface area of 3.0 or greater and 20 or less; and a total degree of acetyl substitution of the cellulose acetate is 0.7 or greater and 3.0 or less.

Since the average particle size of the cellulose acetate particles of the present disclosure is 80 nm or greater and 100 μm or less, the average particle size may be 100 nm or greater, 1 μm or greater, 2 μm or greater, or 4 μm or greater. In addition, the average particle size may be 80 μm or less, 40 μm or less, 20 μm or less, or 14 μm or less. The particles with too large an average particle size are poor in tactile sensation. Alternatively, the particles with too small average particle size may be difficult to produce. Here, the tactile sensation includes, for example, skin feel and tactile sensation of a cosmetic composition containing the particles, in addition to tactile sensation in directly touching the cellulose acetate particles.

The average particle size can be measured using dynamic light scattering. The average particle size (such as in nm and $\mu$m) herein refers to the value of the particle size corresponding to 50% of the integrated scattering intensity in this particle size distribution.

The coefficient of variation of the particle size of the cellulose acetate particles of the present disclosure may be 0% or greater and 60% or less, or may be 2% or greater and 50% or less.

The coefficient of variation (%) of the particle size can be calculated by an equation: (standard deviation of particle size)/(average particle size) $\times$ 100.

Since the sphericity of the cellulose acetate particles of the present disclosure is 0.7 or greater and 1.0 or less, the sphericity is preferably 0.8 or greater and 1.0 or less, and more preferably 0.9 or greater and 1.0 or less. The particles with a sphericity of less than 0.7 are poor in tactile sensation, and, for example, a cosmetic composition containing such particles leads to a reduction in skin feel.

The sphericity can be measured by the following method. The average value of the minor axis length/major axis length ratios of particles selected from an image of the particles observed with a scanning electron microscope (SEM) is defined as the sphericity. The closer to 1 the sphericity is, the closer to the true sphere the particles can be determined to be.

The cellulose acetate particles of the present disclosure have a relative specific surface area (may be hereinafter referred to as RSSA) of 3.0 or greater and 20 or less. The relative specific surface area is preferably 5.0 or greater, more preferably 7.0 or greater, still more preferably 8.5 or greater, yet still more preferably 9.0 or greater, and particularly preferably 10 or greater. Further, the relative specific surface area may be 18 or less. When the relative specific surface area is less than 3.0, truly spherical particles having a smooth surface are formed, the particles having too few or no pore portions. The particles are hardly deformed by an external force applied thereto, and the tactile sensation (particularly, softness) is poor. When the sphericity is more than 20, it is difficult for the particles to maintain high sphericity, and the particles are poor in tactile sensation.

The relative specific surface area (RSSA) is defined by the following relationship: RSSA = specific surface area measurement value/theoretical specific surface area

The theoretical specific surface area is a specific surface area calculated from the measurement results of the particle size distribution, assuming that the particle is a truly spherical particle having a smooth surface, and is defined as follows.

$$\text{Theoretical specific surface area} = (1/d) \, \Sigma \, (P\iota * S\iota/V\iota)$$

d: true specific gravity (constant at 1350 (kg/m$^3$))

$P\iota$: distribution (volume fraction)

$S\iota$; surface area (m$^2$) of one cellulose acetate particle, i.e., a surface area $(4/3)*\pi*(L\iota/2)^3$ of a spherical cellulose acetate particle having a diameter $L\iota$ (m)

$V\iota$: volume (m$^3$) of one cellulose acetate particle, i.e., a volume $4\pi*(L\iota/2)^2$ of a spherical cellulose acetate particle having a diameter $L\iota$ (m)

The specific surface area measurement value is determined according to the BET specific surface area measurement by nitrogen adsorption.

The degree of surface smoothness of the cellulose acetate particles of the present disclosure is preferably 10% or greater and 95% or less, more preferably 50% or greater and 92% or less, and still more preferably 75% or greater and 90% or less. In the case of the particles with too small a degree of surface smoothness, the particles have too many portions (pore portions) corresponding to recesses. When the surface smoothness is too small, it is difficult for the particles to have a truly spherical shape, and a sphericity of 0.7 or greater may not be satisfied. When the sphericity is less than 0.7, the effect of the present disclosure cannot be exhibited. In particular, the tactile sensation is extremely deteriorated. Meanwhile, in the case of the particles with too large a degree of surface smoothness, the particles have too few or no pore portions. The particles are hardly deformed by an external force applied thereto, and the tactile sensation (particularly softness) is poor, as a result of which sufficient oil absorption may not be given.

The degree of surface smoothness can be determined as follows: a scanning electron micrograph of the particles is obtained, recesses and protrusions of the particle surfaces are observed, and the degree of surface smoothness is determined based on the area of recessed portions on the surfaces.

Since the total degree of acetyl substitution of cellulose acetate in the cellulose acetate particles of the present disclosure is 0.7 or greater and 3.0 or less, the total degree of acetyl substitution is preferably 0.7 or greater and less than 2.9, more preferably 1.0 or greater and less than 2.9, still more preferably 1.4 or greater and less than 2.9, particularly preferably 1.8 or greater and less than 2.9, and most preferably 1.6 or greater and less than 2.9.

When the total degree of acetyl substitution of the cellulose acetate is less than 0.7, the water solubility increases and cellulose acetate tends to elute in removing the first thermoplastic polymer and the second thermoplastic polymer from the mixture, in the method for producing cellulose acetate particles to be described later. This may reduce the sphericity of the resulting particles and thus may lead to poor tactile sensation.

The total degree of acetyl substitution of the cellulose acetate can be measured by the following method. First, the total degree of acetyl substitution is the sum of each degree of substitution at position 2-, 3-, and 6- of the glucose ring of the cellulose acetate, and each degree of acetyl substitution at position 2-, 3-, and 6- of the glucose ring of the cellulose acetate in the particles can be measured by NMR according to the method of Tezuka (Tezuka, Carbonydr. Res. 273, 83 (1995)). That is, the free hydroxyl group of a cellulose acetate sample is propionylated with propionic anhydride in pyridine. The resulting sample is dissolved in deuterated chloroform, and the $^{13}$C-NMR spectrum is measured. The carbon signals of the acetyl group appear in the region from 169 ppm to 171 ppm in the order of 2-, 3-, and 6-positions from the high magnetic field; and the carbonyl carbon signals of the propionyl group appear in the region from 172 ppm to 174 ppm in the same order. Each degree of acetyl substitution at the 2-, 3-, and 6-positions of the glucose ring in the original cellulose acetate can be determined from the presence ratio of the acetyl group and the propionyl group at the respective corresponding positions. The degree of acetyl substitution can be analyzed by $^1$H-NMR in addition to $^{13}$C-NMR.

Furthermore, the total degree of acetyl substitution is determined by converting the acetylation degree determined according to the method for measuring the acetylation degree in ASTM: D-817-91 (Testing methods for cellulose acetate, etc.). This is the most common procedure to determine the degree of substitution of cellulose acetate.

$$DS = 162.14 \times AV \times 0.01/(60.052 - 42.037 \times AV \times 0.01)$$

In the above equation, DS is the total degree of acetyl substitution, and AV is the acetylation degree (%). Note that the value of the degree of substitution calculated by the conversion usually has a slight discrepancy from the value measured by NMR described above. When the converted value and the value measured by NMR are different, the value measured by NMR is adopted. In addition, if the value varies among the specific methods of NMR measurement, the value measured by NMR according to the method of Tezuka described above is adopted.

The method for measuring the acetylation degree according to ASTM: D-817-91 (Testing methods for cellulose acetate, etc.) is outlined as follows. First, 1.9 g of dried cellulose acetate is accurately weighed and dissolved in 150 mL of a mixed solution of acetone and dimethyl sulfoxide (a volume ratio of 4:1), then 30 mL of a 1 N sodium hydroxide solution is added, and the cellulose acetate is saponified at 25°C for 2 hours. Phenolphthalein is added as an indicator, and the excess sodium hydroxide is titrated with 1N-sulfuric acid (concentration factor: F). In addition, a blank test is performed in the same manner as described above, and the acetylation degree is calculated according to the following equation.

$$\text{Average acetylation degree } (\%) = \{6.5 \times (B - A) \times F\}/W$$

where A represents the titration volume (mL) of the 1 N sulfuric acid for the sample, B represents the titration volume (mL) of the 1 N sulfuric acid for the blank test, F represents the concentration factor of the 1 N sulfuric acid, and W represents the weight of the sample.

The cellulose acetate particles of the present disclosure may have a bulk specific gravity of 0.1 or greater and 0.9 or less, 0.2 or greater and 0.9 or less, or 0.2 or greater and 0.7 or less. For example, for a cosmetic containing the particles, the higher the bulk specific gravity of the particles, the better the flowability of the cosmetic composition is. The bulk specific gravity can be measured by a method in accordance with JIS K 1201-1.

The oil absorption of the cellulose acetate particles of the present disclosure using linseed oil is preferably 60 ml or greater, more preferably 70 ml or greater, and still more preferably 80 ml or greater per 100 g of the cellulose acetate particles. The oil absorption may be 200 ml or less, and is preferably 100 ml or less, more preferably 90 ml or less. When the oil absorption is 60 ml or greater per 100 g of the cellulose acetate particles, the cellulose acetate particles are particularly excellent in tactile sensation, for example, a cosmetic composition containing the cellulose acetate particles provides improved skin feel. When the oil absorption is more than 200 ml per 100 g of the cellulose acetate particles, the cosmetic composition absorbs the oil content of skin more than necessary, which may cause dryness of the skin. From the viewpoint of preventing excessive dryness of the skin and preparing a cosmetic composition giving good skin feel, the oil absorption may be from 60 ml to 200 ml, from 60 ml to 100 ml, from 60 ml to 90 ml, from 70 ml to 200 ml, from 70 ml to 100 ml, from 70 ml to 90 ml, from 80 ml to 200 ml, from 80 ml to 100 ml, or from 80 ml to 90 ml per 100 g of the cellulose acetate particles.

The oil absorption using linseed oil can be determined by Test methods for pigments, specified in JIS K 5101-13-1: 2004 (ISO 787-5: 1980) -Part 13: Oil absorption -- Section 1: Refined linseed oil method.

The cellulose acetate particles of the present disclosure may contain or need not contain a plasticizer. In the present disclosure, the plasticizer refers to a compound capable of increasing the plasticity of the cellulose acetate. The plasticizer is not particularly limited, and examples include adipate-based plasticizers containing an adipate ester, such as dimethyl adipate, dibutyl adipate, diisostearyl adipate, diisodecyl adipate, diisononyl adipate, diisobutyl adipate, diisopropyl adipate, diethylhexyl, adipate dioctyl adipate, dioctyldodecyl adipate, dicapryl adipate, and dihexyldecyl adipate; citrate-based plasticizers containing a citrate ester, such as acetyl triethyl citrate, acetyl tributyl citrate, isodecyl citrate, isopropyl citrate, triethyl citrate, triethylhexyl citrate, and tributyl citrate; glutarate-based plasticizers containing a glutarate ester, such as diisobutyl glutarate, dioctyl glutarate, and dimethyl glutarate; succinate-based plasticizers containing a succinate ester, such as diisobutyl succinate, diethyl succinate, diethylhexyl succinate, and dioctyl succinate; sebacate-based plasticizers containing a sebacate ester, such as diisoamyl sebacate, diisooctyl sebacate, diisopropyl sebacate, diethyl sebacate, diethylhexyl sebacate, and dioctyl sebacate; glycerin ester-based plasticizers containing a glycerin alkyl ester, such as triacetin, diacetin, and monoacetin; neopentyl glycol; phthalate-based plasticizers containing a phthalate ester, such as ethyl phthalate, methyl phthalate, diaryl phthalate, diethyl phthalate, diethylhexyl phthalate, dioctyl phthalate, dibutyl phthalate, and dimethyl phthalate; and phosphate-based plasticizers containing a phosphate ester, such as trioleil phosphate, tristearyl phosphate, and tricetyl phosphate. In addition, examples thereof also include di-2-methoxyethyl phthalate, dibutyl tartrate, ethyl 0-benzoylbenzoate, ethyl phthalyl ethyl glycolate (EPEG), methyl phthalyl ethyl glycolate (MPEG), N-ethyl toluene sulfonamide, 0-cresyl p-toluenesulfonate, triethyl phosphate (TEP), triphenyl phosphate (TPP), and tripropionin. These plasticizers may be used alone, or two or more of the plasticizers may be used in combination.

Among the plasticizers, the plasticizer is preferably at least one selected from the group consisting of citrate-based plasticizers containing a citrate ester, such as triethyl citrate, acetyl triethyl citrate, and acetyl tributyl citrate; glycerin ester-based plasticizers containing a glycerin alkyl ester, such as triacetin, diacetin, and monoacetin; and phthalate-based plasticizers, such as ethyl phthalate and methyl phthalate; more preferably at least one selected from the group consisting of triethyl citrate, acetyl triethyl citrate, acetyl tributyl citrate, triacetin, diacetin, diisononyl adipate, and diethyl phthalate; still more preferably at least one selected from the group consisting of acetyl triethyl citrate, triacetin, diacetin, and diethyl phthalate; and particularly preferably at least one selected from the group consisting of acetyl triethyl citrate and triacetin. Note that a phthalate-based plasticizer must be used with care because of concerns about similarity to environmental hormones.

When the cellulose acetate particles contain a plasticizer, the content of the plasticizer included in the cellulose acetate particles is not particularly limited. For example, the content of the plasticizer may be more than 0 parts by weight and 67 parts by weight or less, 2 parts by weight or greater and 67 parts by weight or less, 11 parts by weight or greater and 43 parts by weight or less, or 18 parts by weight or greater and 25 parts by weight or less relative to 100 parts by weight of the cellulose acetate.

The content rate of the plasticizer in the cellulose acetate particles is determined by $^1$H-NMR measurement.

The cellulose acetate particles of the present disclosure have excellent biodegradability. The biodegradation rate is preferably 40 wt.% or greater, more preferably 50 wt.% or greater, and still more preferably 60 wt.% or greater within 30 days.

The biodegradation rate can be measured by a method using activated sludge in accordance with JIS K6950.

The cellulose acetate particles of the present disclosure can be produced by a production method described later.

The cellulose acetate particles of the present disclosure are excellent in biodegradability, tactile sensation, and oil absorbability, and thus can be suitably used, for example, in cosmetic compositions. In a cosmetic composition containing the cellulose acetate particles of the present disclosure, the oil absorption of the cellulose acetate particles is high, and thus the cellulose acetate particles can absorb sebum and prevent makeup from coming off. Further, the cellulose acetate particles of the present disclosure have a high sphericity, are likely to be deformed by an external force applied thereto, and are excellent in softness. Accordingly, the cellulose acetate particles can improve the tactile sensation of the cosmetic composition. Additionally, since the cellulose acetate particles of the present disclosure have many pores, it is easy to cause the pores to carry a functional agent or the like, and the cellulose acetate particles can be suitably used as functional particles.

Examples of the cosmetic composition include foundation, such as liquid foundation and powder foundation; concealers; sunscreens; makeup bases; lipsticks and lipstick bases; Oshiroi face powders, such as body powders, solid face powders, and face powders; solid powder eye shadows; wrinkle masking creams; and skin and hair external preparations mainly for cosmetic purposes, such as skin care lotions; and the dosage form is not limited. The dosage form may be any of a liquid preparation, such as an aqueous solution, a milky lotion, and a suspension; a semi-solid preparation, such as a gel and a cream; or a solid preparation, such as a powder, a granule, and a solid. In addition, the dosage form may be an emulsion preparation, such as a cream and a milky lotion; an oil gel preparation, such as a lipstick; a powder preparation, such as a foundation; an aerosol preparation, such as a hair styling agent; or the like.

Method for Producing Cellulose Acetate Particles

A method for producing cellulose acetate particles of the present disclosure is as follows. A method for producing cellulose acetate particles includes: mixing cellulose acetate having a total degree of acetyl substitution of 0.7 or greater and 3.0 or less, a plasticizer, a first thermoplastic polymer, and a second thermoplastic polymer to form a mixture of cellulose acetate containing the plasticizer, the first thermoplastic polymer, and the second thermoplastic polymer; melt-kneading the mixture at 200°C or higher and 280°C or lower; and removing the first thermoplastic polymer and the second thermoplastic polymer from the melt-kneaded mixture, in which when SPa represents an SP value of the cellulose acetate, SPb represents an SP value of the first thermoplastic polymer, and SPc represents an SP value of the second thermoplastic polymer, SPa, SPb, and SPc satisfy the following relationship:

$$0.1 \leq |\, SPc - SPa \,| \,/\, |\, SPb - SPa \,| \leq 0.9.$$

Formation of Mixture of Cellulose Acetate

Cellulose acetate having a total degree of acetyl substitution of 0.7 or greater and 3.0 or less, a plasticizer, a first thermoplastic polymer, and a second thermoplastic polymer are mixed in the formation of a mixture of cellulose acetate containing the plasticizer, the first thermoplastic polymer, and the second thermoplastic polymer.

The cellulose acetate having a total degree of acetyl substitution of 0.7 or greater and 3.0 or less can be produced by a well-known method for producing cellulose acetate. Examples of such a production method include what is called an acetic acid method in which acetic anhydride is used as an acetylating agent, acetic acid as a diluent, and sulfuric acid as a catalyst. The basic processes of the acetic acid method include: (1) pretreatment including grinding/disintegrating a pulp raw material (soluble pulp) having a relatively high $\alpha$-cellulose content and then spraying acetic acid and mixing them; (2) acetylation including reacting the pretreated pulp from (1) with a mixed acid containing acetic anhydride, acetic acid, and an acetylation catalyst (e.g., sulfuric acid); (3) aging including hydrolyzing cellulose acetate to form cellulose acetate having a desired acetylation degree; and (4) post-treatment including precipitating the cellulose acetate to separate it from the reaction solution after completion of the hydrolysis reaction, then purifying, stabilizing, and drying the cellulose acetate.

Since the total degree of acetyl substitution of the cellulose acetate is 0.7 or greater and 3.0 or less, the total degree of acetyl substitution is preferably 0.7 or greater and less than 2.9, more preferably 1.0 or greater and less than 2.9, still more preferably 1.4 or greater and less than 2.9, particularly preferably 1.8 or greater and less than 2.9, and most preferably 1.6 or greater and less than 2.9. The total degree of acetyl substitution can be adjusted by adjusting the conditions of aging (conditions, such as time and temperature).

Any plasticizer having a plasticizing effect in melt-extruding cellulose acetate can be used without particular limitation. Specifically, the plasticizers exemplified as a plasticizer contained in the cellulose acetate particles may be used alone or two or more of the plasticizers may be used in combination.

Among the plasticizers exemplified above, the plasticizer is preferably at least one selected from the group consisting of citrate-based plasticizers containing a citrate ester, such as triethyl citrate, acetyl triethyl citrate, and acetyl tributyl citrate; glycerin ester-based plasticizers containing a glycerin alkyl ester, such as triacetin, diacetin, and monoacetin; adipate-based plasticizers, such as diisononyl adipate; and phthalate-based plasticizers, such as ethyl phthalate and methyl phthalate; more preferably at least one selected from the group consisting of triethyl citrate, acetyl triethyl citrate, acetyl tributyl citrate, triacetin, diacetin, diisononyl adipate, and diethyl phthalate; still more preferably at least one selected from the group consisting of acetyl triethyl citrate, triacetin, diacetin, and diethyl phthalate; and particularly preferably at least one selected from the group consisting of acetyl triethyl citrate and triacetin. Note that a phthalate-based plasticizer must be used with care because of concerns about similarity to environmental hormones.

The blending amount of the plasticizer may be more than 0 parts by weight and 67 parts by weight or less, 2 parts by weight or greater and 67 parts by weight or less, 11 parts by weight or greater and 43 parts by weight or less, or 18 parts by weight or greater and 25 parts by weight or less relative to 100 parts by weight of the cellulose acetate. Too small blended amount of the plasticizer would tend to reduce the sphericity of the resulting cellulose acetate particles, and too large blended amount of the plasticizer would fail to maintain the shape of the particles, tending to reduce the sphericity.

The first thermoplastic polymer and the second thermoplastic polymer can be used without any particular limitation as long as they satisfy the relationship below.

When SPa represents an SP value of the cellulose acetate, SPb represents an SP value of the first thermoplastic polymer, and SPc represents an SP value of the second thermoplastic polymer, SPa, SPb, and SPc satisfy the following relationship:

$$0.1 \leq | \, SPc - SPa \, | \, / \, | \, SPb - SPa \, | \leq 0.9.$$

The thermoplastic polymer in the present specification is not particularly limited as long as it is a polymer having a wide range of thermoplasticity. Both the first thermoplastic polymer and the second thermoplastic polymer preferably have water solubility, in other words, are preferably water-soluble polymers. Here, the water-soluble polymer means that an insoluble content is less than 50 wt.% when 1 g of the polymer is dissolved in 100 g of water at 25°C. Further, the "polymer" is defined as a compound having a structure formed by repeatedly bonding one or two or more of constituent units. In the present specification of the present application, a compound having a weight-average molecular weight of 10000 or greater is referred to as a "polymer".

Examples of the first thermoplastic polymer or the second thermoplastic polymer include polyvinyl alcohol, polyethylene glycol, sodium polyacrylate, polyvinylpyrrolidone, polypropylene oxide, polyglycerol, polyethylene oxide, polyvinyl acetate, modified starch, thermoplastic starch, methylcellulose, ethylcellulose, hydroxyethyl cellulose, and hydroxypropylcellulose. Further, the thermoplastic starch can be prepared by a well-known method. For example, reference can be made to JP H06-006307 B, WO 92/04408, etc., and more specifically, for example, a thermoplastic starch prepared by mixing approximately 20% of glycerin as a plasticizer to tapioca starch and kneading them with a twin-screw extruder can be used.

The first thermoplastic polymer preferably contains at least one selected from the group consisting of polyvinyl alcohol, sodium polyacrylate, polyvinylpyrrolidone, and thermoplastic starch, and more preferably contains at least one selected from the group consisting of polyvinyl alcohol and thermoplastic starch. Further, the weight-average molecular weight of polyvinyl alcohol is preferably 500 or greater and 50000 or less.

The second thermoplastic polymer may be a thermoplastic polymer that satisfies $0.1 \leq | \, SPc - SPa \, | \, / \, | \, SPb - SPa \, | \leq 0.9$. Preferred is a thermoplastic polymer that satisfies $0.2 < | \, SPc - SPa \, | \, / \, | \, SPb - SPa \, | < 0.8$. When this value is $| \, SPc - SPa \, | \, / \, | \, SPb - SPa \, | < 0.1$ or $| \, SPc - SPa \, | \, / \, | \, SPb - SPa \, | > 0.9$, the size of pores formed in the resulting cellulose acetate particles is small, the number of pores is also small, the relative specific surface area (RSSA) is reduced, and the tactile sensation is poor. Thus, this is not preferred.

The second thermoplastic polymer is preferably polyethylene glycol.

When polyvinyl alcohol, thermoplastic starch, or modified starch is used as the first thermoplastic polymer, it is particularly preferable to use polyethylene glycol as the second thermoplastic polymer. This is because polyvinyl alcohol, thermoplastic starch, modified starch, and polyethylene glycol are all water-soluble and thermoplastic. Further, the weight-average molecular weight of polyethylene glycol is preferably 500 or greater and 50000 or less.

Here, the weight-average molecular weight (Mw) is a value obtained by multiplying individual molecules by their molecular weights and taking the weighted average, and is determined by gel permeation chromatography (GPC).

The blending amount of the first thermoplastic polymer is preferably 110 parts by weight or greater and 15000 parts by weight or less, more preferably 180 parts by weight or greater and 1200 parts by weight or less, still more preferably 200 parts by weight or greater and 800 parts by weight or less relative to 100 parts by weight of the cellulose acetate. When the blending amount is less than 110 parts by weight, cellulose acetate particles having poor sphericity, i.e., having a non-spherical modified shape, may be produced. When the blending amount is more than 15000 parts by weight, the particle size of the resulting cellulose acetate particles may be too small.

The blending amount of the second thermoplastic polymer is preferably 1 part by weight or greater and 1500 parts by weight or less, more preferably 2 parts by weight or greater and 150 parts by weight or less, still more preferably 3 parts by weight or greater and 100 parts by weight or less relative to 100 parts by weight of the cellulose acetate. When the blending amount is less than 1 part by weight, sufficient pores are not formed in the resulting cellulose acetate particles, and the oil absorption may become insufficient.

Mixing of the cellulose acetate and the plasticizer, or mixing of the cellulose acetate, the plasticizer, the first thermoplastic polymer, and the second thermoplastic polymer can be performed in a dry or wet manner using a mixer such as a Henschel mixer. In using a mixer, such as a Henschel mixer, the temperature in the mixer may be a temperature at which the cellulose acetate does not melt, for example, in a range of not lower than 20°C and lower than 200°C.

Alternatively, mixing of the cellulose acetate and the plasticizer, or mixing of the cellulose acetate, the plasticizer, the first thermoplastic polymer, and the second thermoplastic polymer may be performed by melt-kneading. Furthermore, the melt-kneading may be performed in combination with mixing using a mixer, such as a Henschel mixer, and in this case, the melt-kneading is preferably performed after mixing in temperature conditions in a range of not lower than 20°C and lower than 200°C using a mixer, such as a Henschel mixer. The plasticizer and the cellulose acetate, or the plasticizer, the first thermoplastic polymer, the second thermoplastic polymer, and the cellulose acetate become more uniform and mixed well in a short period of time, thus increasing the sphericity of the cellulose acetate particles that are finally prepared and improving the tactile sensation and touch feeling of the particles.

The melt-kneading is preferably performed by heating and mixing with an extruder. The kneading temperature (cylinder temperature) of the extruder may be in a range of 200°C to 230°C. The melt-kneading performed even at temperatures in this range can plasticize the cellulose acetate and provide a uniform kneaded product. The melt-kneading performed at too low temperatures may reduce the sphericity of the resulting particles, thus reducing the tactile sensation and the touch feeling. The melt-kneading performed at too high temperatures may cause deterioration or coloration of the kneaded product due to heat and may reduce the viscosity of the melted material, thus failing to sufficiently knead the resin in a twin-screw extruder.

The melting point of the cellulose acetate depends on the degree of substitution but is approximately from 230°C to 280°C and is close to the decomposition temperature of the cellulose acetate. Thus, melt-kneading is typically difficult in this temperature range, but because the cellulose acetate (flakes) impregnated with the plasticizer can reduce the plasticizing temperature. The kneading temperature (cylinder temperature) may be, for example, 200°C in using a twin-screw extruder. The kneaded product may be extruded in a strand shape and formed into a pellet form by hot cutting or the like. The die temperature in this case may be approximately 220°C.

Melt-kneading of Mixture of Cellulose Acetate

In the present step, the mixture is melt-kneaded at 200°C or higher and 280°C or lower.

The mixture can be kneaded by an extruder such as a twin-screw extruder. Further, the temperature of the kneading refers to the cylinder temperature.

The mixture of cellulose acetate may be extruded into a string shape from a die attached to the tip of an extruder, such as a twin-screw extruder, and then cut into pellets. At this time, the die temperature may be not lower than 220°C and not higher than 300°C.

Removal of First Thermoplastic Polymer and Second Thermoplastic Polymer

The removal of the first thermoplastic polymer and the second thermoplastic polymer from the melt-kneaded mixture will be described.

The method of removing the first thermoplastic polymer and the second thermoplastic polymer is not particularly limited as long as the first thermoplastic polymer and the second thermoplastic polymer can be removed from the melt-kneaded mixture by dissolution or the like. Examples thereof include a method of removing the first thermoplastic polymer and the second thermoplastic polymer from the mixture by dissolving the thermoplastic polymers in water; an alcohol such as methanol, ethanol, or isopropanol; or a solvent such as a mixed solution thereof. Specifically, examples thereof include a method of removing the first thermoplastic polymer and the second thermoplastic polymer from the mixture, such as by mixing the mixture and the solvent and filtering the mixture to take out the filtrate.

In removing the first thermoplastic polymer and the second thermoplastic polymer from the mixture, the plasticizer may be removed, or need not be removed from the mixture together with the first thermoplastic polymer and the second thermoplastic polymer. Thus, the resulting cellulose acetate particles may or may not contain a plasticizer.

Regarding the mixing ratio of the mixture and the solvent, the mixture is preferably 0.01 wt.% or greater and 20 wt.% or less, more preferably 2 wt.% or greater and 15 wt.% or less, and still more preferably 4 wt.% or greater and 13 wt.% or less relative to the total weight of the mixture and the solvent. When the mixing ratio of the mixture is more than 20 wt.%, dissolution of the first and second thermoplastic polymers becomes insufficient and the first and second thermoplastic polymers cannot be removed by washing, or it may be difficult to separate the cellulose acetate particles not dissolved in the solvent from the first and second thermoplastic polymers dissolved in the solvent by an operation such as filtration or centrifugal separation.

The mixing temperature of the mixture and the solvent is preferably 0°C or higher and 200°C or lower, more preferably 20°C or higher and 110°C or lower, and still more preferably 40°C or higher and 80°C or lower. At temperatures lower than 0°C, the first thermoplastic polymer and the second thermoplastic polymer may not be sufficiently dissolved and it may be difficult to remove them by washing, whereas, at temperatures higher than 200°C, deformation, aggregation, or the like of the particles may occur, and it may be difficult to take out the particles while maintaining the desired shape of the particles.

The mixing time of the mixture and the solvent is not particularly limited, and may be appropriately adjusted, but may be, for example, 0.5 hours or longer, 1 hour or longer, 3 hours or longer, 5 hours or longer, or may be 6 hours or shorter.

In addition, the method of mixing is not limited as long as the method can dissolve the first thermoplastic polymer and the second thermoplastic polymer, but, for example, use of a stirring device, such as an ultrasonic homogenizer or a Three-One Motor, can efficiently remove the first thermoplastic polymer and the second thermoplastic polymer from the mixture even at room temperature.

For example, when a Three-One Motor is used as the stirring device, the rotation speed during mixing the mixture and the solvent may be, for example, 5 rpm or greater and 3000 rpm or less. This enables the first thermoplastic polymer and the second thermoplastic polymer to be efficiently removed from the mixture. In addition, this also efficiently removes the plasticizer from the mixture.

Each aspect disclosed in the present specification can be combined with any other feature disclosed herein.

Examples

Hereinafter, the present disclosure will be specifically described with reference to examples, but the technical scope of the present disclosure is not limited by these examples. Note that each of the configurations, combinations thereof, and the like in each of the embodiments are an example, and various additions, omissions, substitutions, and other changes may be made as appropriate without departing from the spirit of the present disclosure.

Example A-1

First, 100 parts by weight of cellulose diacetate (available from Daicel Corporation: total degree of acetyl substitution DS = 2.4, SP value: 24 ($MPa^{1/2}$)) and 25 parts by weight of triacetin as a plasticizer were blended in a dry state, dried at 80°C for 12 hours or longer, further stirred and mixed using a Henschel mixer, and a mixture of the cellulose acetate and the plasticizer was prepared. The resulting mixture was fed to a twin-screw extruder (PCM30 available from Ikegai Corp., a cylinder temperature of 200°C, a die temperature of 220°C), melt-kneaded, extruded, pelletized, and a kneaded product was formed.

100 parts by weight of the pellets of the resulting kneaded product, 271 parts by weight of polyvinyl alcohol (PVA available from The Nippon Synthetic Chemical Industry Co., Ltd.: melting point: 190°C, degree of saponification: 99.1%, SP value: 34 ($MPa^{1/2}$)) as the first thermoplastic polymer, and 21 parts by weight of polyethylene glycol (PEG, SP value: 20 ($MPa^{1/2}$)) as the second thermoplastic polymer were dry-blended. Then, the mixture was fed to a twin-screw extruder (PCM30 available from Ikegai Corp., cylinder temperature: 220°C, die temperature: 220°C), and extruded to form a mixture of cellulose acetate.

The resulting mixture of cellulose acetate was combined with pure water (a solvent) to give a concentration of not more than 5 wt.% (weight of mixture/(weight of mixture + weight of pure water) $\times$ 100), and the mixture was stirred using a Three-One Motor (BL-3000 available from Shinto Scientific Co., Ltd.) at a rotation speed of 100 rpm, at a temperature of 80°C for 3 hours. The solution after stirring was filtered off with filter paper (No. 5A available from ADVANTEC), and the filtrate was taken out. The resulting filtrate was prepared using pure water again to give a concentration of the mixture of not more than 5 wt.%, the mixture was further stirred at a rotation speed of 100 rpm, at a temperature of 80°C for 3 hours, and the solution was filtered off to take out the filtrate. This operation was repeated three or more times, and cellulose acetate particles were obtained.

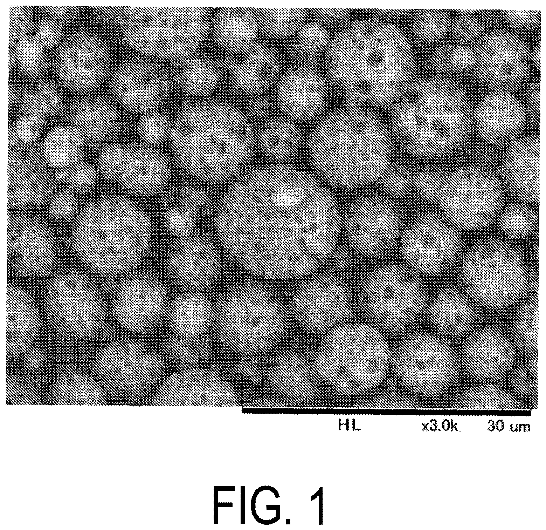

The average particle size, coefficient of variation of particle size, sphericity, oil absorption, degree of surface smoothness, bulk specific gravity, and RSSA of the resulting cellulose acetate particles were determined, and the biodegradability and tactile sensation were evaluated. The results are shown in Table 1. Note that the measurement or evaluation of the average particle size, coefficient of variation of particle size, sphericity, oil absorption, degree of surface smoothness, bulk specific gravity, RSSA, biodegradability, and tactile sensation were measured or evaluated by the following methods. The scanning electron microscope (SEM) image is as shown in FIG. 1-3. Lengths of scale bars in FIGS. 1 to 3 are 30 $\mu$m in the SEM image magnified 3000 times, 20 $\mu$m in the SEM image magnified 5000 times, and 5.00 $\mu$m in the SEM image magnified 6000 times, respectively.

Average particle size and coefficient of variation of particle size

The average particle size was measured using dynamic light scattering. First, the sample was combined with pure water to adjust the concentration to approximately 100 ppm, and a pure water suspension was prepared using an ultrasonic vibrating device. Then, the particle size volume distribution was determined by laser diffraction ("Laser Diffraction/Scattering Particle Size Distribution Measuring Device LA-960" available from Horiba Ltd., ultrasonic treatment for 15 minutes, a refractive index (1.500, medium (water; 1.333)), and the average particle size was measured. The average particle size (in nm, $\mu$m, etc.) herein was the value of the particle size corresponding to 50% of the integrated scattering intensity in the particle size volume distribution. In addition, the coefficient of variation (%) of the particle size was calculated by an equation: standard deviation of particle size/average particle size $\times$ 100.

Sphericity

Using an image of particles observed with a scanning electron microscope (SEM), the major axis length and the minor axis length of 30 randomly selected particles were measured to determine the (minor axis length)/(major axis length) ratio of each particle, and the average value of the (minor axis length)/(major axis length) ratios was taken as the sphericity.

Oil absorption

The oil absorption was determined by Test methods for pigments, specified in JIS K 5101-13-1: 2004 (ISO 787-5: 1980) -Part 13: Oil absorption -- Section 1: Refined linseed oil method.

Degree of surface smoothness

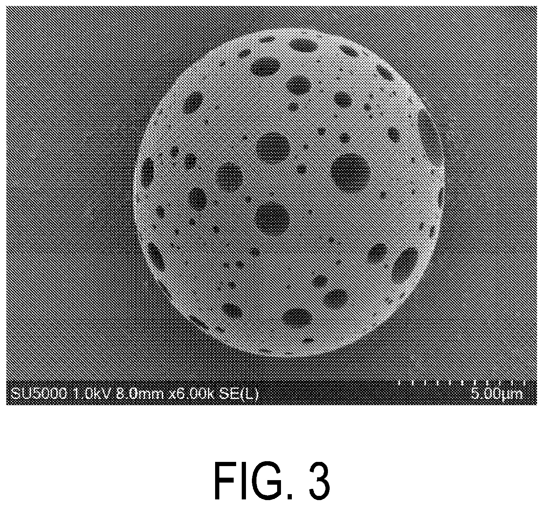

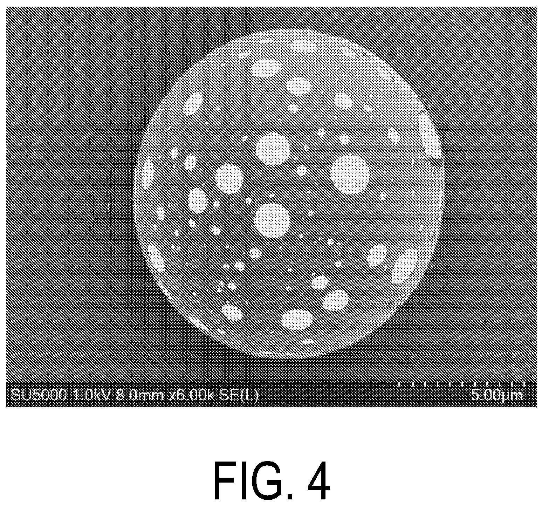

A scanning electron micrograph of the particles was taken at a magnification of 2500 to 6000 times (see FIG. 3 for an example of a micrograph of the cellulose acetate particles in Example A-1, taken with "SU 5000": trade name, available from Hitachi High-Technologies Corporation), and the image was binarized using an image processor WinrOOF (available from Mitani Corporation) (see FIG. 4 for the binarized image of the micrograph of FIG. 3). A region including the center and/or near the center of one particle was randomly selected from the binarized image. The area ratio of a portion (shade portion) corresponding to a recess of unevenness in the region was calculated, and the degree of surface smoothness (%) of one of the particles was calculated by the following formula:

$$\text{Degree of surface smoothness of one particle } (\%) = (1 - \text{area ratio of recesses}) \times 100$$

$$\text{Area ratio of recesses} = \text{area of recessed portions in the any area/the any área}$$

The average value of the degree of surface smoothness of randomly selected 10 particle samples, that is, from n1 to 10, was taken as the degree of surface smoothness (%). The higher this numerical value, the higher the degree of surface smoothness is. Note that the region used for calculating the area ratio may be any areas each smaller than the particle including the center and/or near the center of one particle. In addition, the size of the area may be 5 $\mu$m square for the particle with a diameter of 15 $\mu$m.

bulk specific gravity

The bulk specific gravity was measured according to "JIS K 1201-1".

Relative specific surface area: RSSA

A specific surface area calculated from the measurement results of the particle size distribution, assuming that the particle was a truly spherical particle having a smooth surface, was defined as "theoretical specific surface area", and the specific surface area measured by the BET method was defined as "specific surface area measurement value ", the following relationship was established: relative specific surface area (RSSA) = specific surface area measurement value/theoretical specific surface area.

The method of measuring the specific surface area by the BET method is as follows. The specific surface area using the BET specific surface area measurement by nitrogen adsorption can be determined by previously evacuating ports holding samples in a MasterPrep degasser (available from Quantachrome Instruments) under heating at a temperature of 100°C for about 1 hour, then measuring nitrogen adsorption at about 7 points in a relative pressure range of 0.05 to 0.28 by a nitrogen gas adsorption method using a specific surface area measuring device ("Autosorb iQ Station 2" available from Quantachrome Instruments), and calculating the specific surface areas using the BET method.

Biodegradability

Biodegradability was evaluated by biodegradation rate. The biodegradation rate was measured by a method using activated sludge in accordance with JIS K6950. The activated sludge was obtained from a municipal sewage-treatment plant. About 300 mL of a supernatant (activated sludge concentration: about 360 ppm) obtained by allowing the activated sludge to stand for approximately 1 hour was used per culture bottle. The measurement was started when 30 mg of the sample was stirred in the supernatant, and then the sample was measured every 24 hours until after 720 hours, that is until after 30 days, a total of 31 times. Details of the measurement are as follows. The biochemical oxygen demand (BOD) in each culture bottle was measured using a Coulometer OM3001 available from Ohkura Electric Co., Ltd. The percentage of the biochemical oxygen demand (BOD) to the theoretical biochemical oxygen demand (BOD) in complete degradation based on the chemical composition of each sample was taken as the biodegradation rate (wt.%), and the biodegradability was evaluated as follows.

Excellent: more than 60 wt.%. Good: 40 wt.% or greater and 60 wt.% or less.
Marginal: 10 wt.% or greater and less than 40 wt.%. Poor: less than 10 wt.%.

Tactile sensation

Sensory evaluation was performed according to a panel test by 20 panelists for the tactile sensation of the particles. Panelists were instructed to touch the particles to evaluate comprehensively softness, smoothness and moist feeling, on a scale with a maximum score of 5 points according to the following criteria, and an average score from 20 panelists was calculated.
Good: 5. Slightly good: 4. Average: 3. Slightly poor: 2. Poor: 1.

Scanning electron microscope (SEM) image

Scanning electron microscope (SEM) images were taken at magnifications of 3000 times, 5000 times, and 6000 times. A scanning electron microscope (trade name "TM 3000" available from Hitachi High-Technologies Corporation) was used to take images at magnifications of 3000 times and 5000 times, and a scanning electron microscope (trade name "SU 5000" available from Hitachi High-Technologies Corporation) was used to take images at a magnification of 6000 times.

Examples A-2, A-3, A-5, and A-7 to A-12

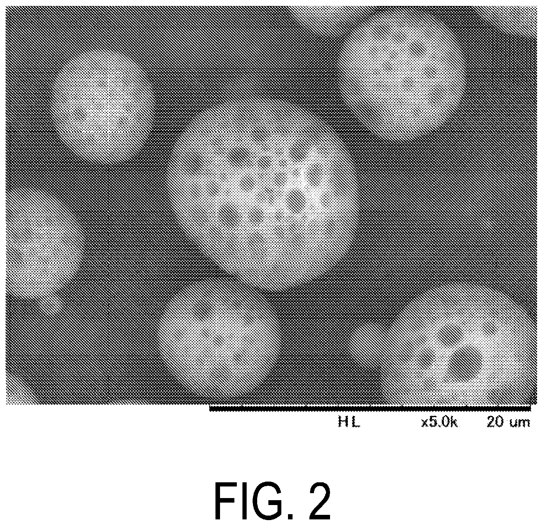

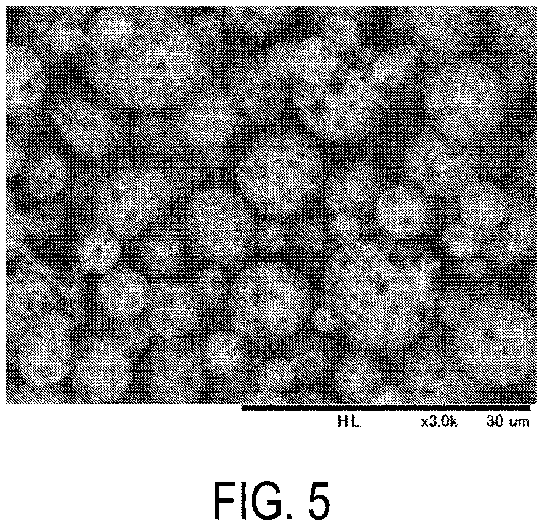

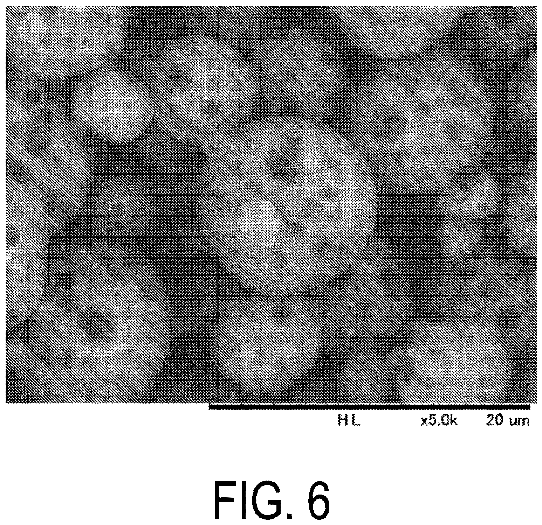

Cellulose acetate particles were prepared in the same manner as in Example A-1 except that the type and blending amount of each of the plasticizer, the first thermoplastic resin, and the second thermoplastic resin were changed as shown in Table 1. Each physical property of the resulting cellulose acetate particles was evaluated according to the measurement methods described above. The results are shown in Table 1. In addition, the scanning electron microscope (SEM) images of Example A-1 are shown in FIG. 1 (magnification: 3000), FIG. 2 (magnification: 5000), and FIG. 3 (magnification: 6000), respectively. The SEM images of Example A-12 are shown in FIG. 5 (magnification: 3000) and FIG. 6 (magnification: 5000), respectively.

Examples A-4 and A-6

In Example A-4, the cellulose acetate was replaced with cellulose diacetate (available from Daicel Corporation, total degree of acetyl substitution DS = 2.8, SP value: 22.6 (MPa$^{1/2}$)). In Example A-6, the cellulose acetate was replaced with cellulose diacetate (available from Daicel Corporation, total degree of acetyl substitution DS = 1.8, SP value: 26 (MPa$^{1/2}$)). Further, cellulose acetate particles were prepared in the same manner as in Example A-1 except that the type and blending amount of each of the plasticizer, the first thermoplastic resin, and the second thermoplastic resin were changed as shown in Table 1. Each physical property of the resulting cellulose acetate particles was evaluated according to the measurement methods described above. The results are shown in Table 1.

Comparative Examples A-1 to A-3, A-5, A-7 to A-11

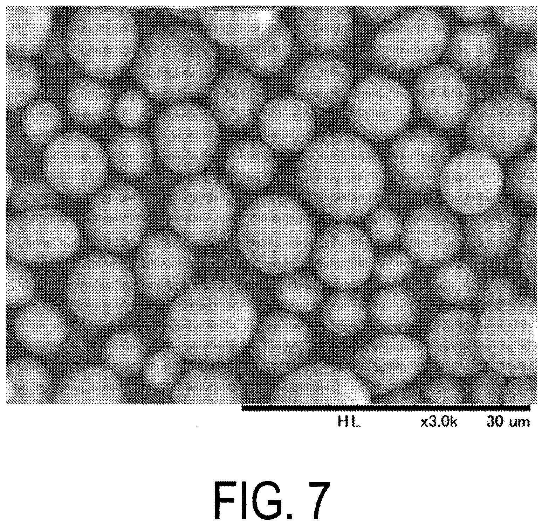

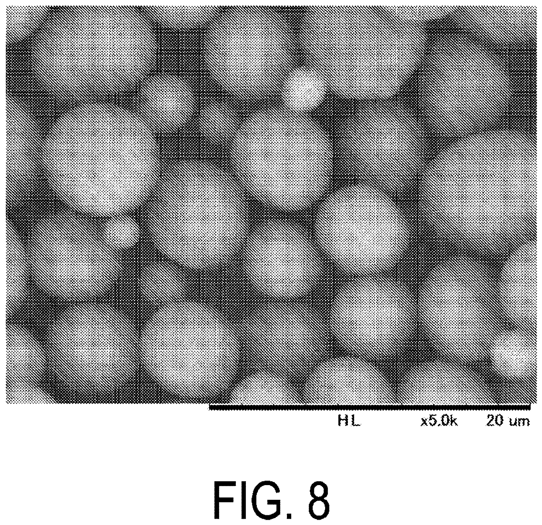

Cellulose acetate particles were prepared in the same manner as in Example A-1 except that the type and blending amount of each of the plasticizer and the first thermoplastic resin were changed as shown in Table 2, and the second thermoplastic resin was not added. Each physical property of the resulting cellulose acetate particles was evaluated according to the measurement methods described above. The results are shown in Table 2. The scanning electron microscope (SEM) images of Comparative Example A-1 are shown in FIG. 7 (3000 times) and FIG. 8 (5000 times).

Comparative Example A-4 and Comparative Example A-6

Cellulose acetate particles of Comparative Example A-4 were prepared in the same manner as in Example A-1 except that the cellulose acetate was replaced with cellulose diacetate (available from Daicel Corporation, total degree of acetyl substitution DS = 2.8, SP value: 22.6 (MPa$^{1/2}$)), the type and blending amount of each of the plasticizer and the first thermoplastic resin were changed as shown in Table 2, and the second thermoplastic resin was not added. Cellulose acetate particles of Comparative Example A-6 were prepared in the same manner as in Example A-1 except that the cellulose acetate was replaced with cellulose diacetate (available from Daicel Corporation, total degree of acetyl substitution DS = 1.8, SP value: 26 (MPa$^{1/2}$)), the type and blending amount of each of the plasticizer and the first thermoplastic resin were changed as shown in Table 2, and the second thermoplastic resin was not added. Each physical property of the resulting cellulose acetate particles was evaluated according to the measurement methods described above. The results are shown in Table 2.

Comparative Examples A-12 to 15

Cellulose acetate particles were prepared in the same manner as in Example A-1 except that the type and blending amount of each of the first thermoplastic resin and the second thermoplastic resin were changed as shown in Table 3. Each physical property of the resulting cellulose acetate particles was evaluated according to the measurement methods described above. The results are shown in Table 3.

[Table 1]

| | | Example A-1 | Example A-2 | Example A-3 | Example A-4 | Example A-5 | Example A-6 | Example A-7 | Example A-8 | Example A-9 | Example A-10 | Example A-11 | Example A-12 |
|---|---|---|---|---|---|---|---|---|---|---|---|---|---|
| Total degree of acetyl substitution (DS) | | 2.4 | 2.4 | 2.4 | 2.8 | 2.4 | 1.8 | 2.4 | 2.4 | 2.4 | 2.4 | 2.4 | 2.4 |
| Plasticizer | Type | Triacetin | Acetyl triethyl citrate | Triacetin | Diethyl phthalate | Acetyl triethyl citrate | Diacetin | Triacetin | Triacetin | Triacetin | Triacetin | Triacetin | Triacetin |
| | Blending amount (part by weight) | 25 | 25 | 25 | 25 | 25 | 25 | 20 | 25 | 25 | 25 | 25 | 25 |
| First thermoplastic resin | Type | PVA | PVA | PVA | PVA | PVA | PVA | PVA | PVA | PVA | PVA | Thermoplastic starch | PVA |
| | Blending amount (part by weight) | 271 | 271 | 271 | 271 | 271 | 271 | 261 | 271 | 271 | 271 | 271 | 283 |
| Second thermoplastic resin | Type | PEG | PEG | PEG | PEG | PEG | PEG | PEG | PEG | PEG | PEG | PEG | PEG |
| | Blending amount (part by weight) | 21 | 21 | 21 | 21 | 21 | 21 | 20 | 21 | 21 | 21 | 21 | 8 |
| Cellulose acetate particles | Average particle size | 13.3 μm | 210 nm | 20 μm | 27 μm | 7.2 μm | 10.1 μm | 40 μm | 13.8 μm | 11.2 μm | 14.1 μm | 13.8 μm | 10.9 μm |
| | Coefficient of variation of particle size | 36% | 40% | 38% | 40% | 37% | 39% | 38% | 44% | 36% | 37% | 39% | 34% |
| | Sphericity | 0.95 | 0.94 | 0.96 | 0.95 | 0.96 | 0.96 | 0.96 | 0.83 | 0.98 | 0.97 | 0.95 | 0.96 |
| | Oil absorption (ml/100 g) | 72 | 86 | 70 | 68 | 77 | 75 | 66 | 71 | 73 | 71 | 72 | 74 |
| | Degree of surface smoothness | 80% | 75% | 85% | 83% | 86% | 87% | 78% | 80% | 82% | 85% | 83% | 80% |

(continued)

| | | Example A-1 | Example A-2 | Example A-3 | Example A-4 | Example A-5 | Example A-6 | Example A-7 | Example A-8 | Example A-9 | Example A-10 | Example A-11 | Example A-12 |
|---|---|---|---|---|---|---|---|---|---|---|---|---|---|
| | bulk specific gravity | 0.50 | 0.43 | 0.46 | 0.43 | 0.47 | 0.49 | 0.50 | 0.48 | 0.51 | 0.46 | 0.48 | 0.50 |
| | Theoretical specific surface area ($m^2$/g) | 0.35 | 0.03 | 0.24 | 0.18 | 0.64 | 0.46 | 0.12 | 0.34 | 0.42 | 0.33 | 0.34 | 0.43 |
| | BET specific surface area ($m^2$/g) | 3.53 | 0.30 | 1.89 | 2.23 | 7.26 | 5.47 | 1.24 | 2.66 | 3.62 | 3.10 | 3.03 | 5.64 |
| | RSSA | 10 | 11.2 | 7.9 | 12.4 | 11.4 | 11.9 | 10 | 7.8 | 8.7 | 9.3 | 8.9 | 13.2 |
| | Biodegradability | Excellent | Excellent | Excellent | Marginal | Excellent | Excellent | Excellent | Excellent | Excellent | Excellent | Excellent | Excellent |
| | Tactile sensation | 4.4 | 4 | 4.2 | 3.9 | 4.2 | 4.4 | 4 | 3.9 | 4.2 | 4.4 | 4.1 | 4.5 |

[Table 2]

| | | Comparative Example A-1 | Comparative Example A-2 | Comparative Example A-3 | Comparative Example A-4 | Comparative Example A-5 | Comparative Example A-6 | Comparative Example A-7 | Comparative Example A-8 | Comparative Example A-9 | Comparative Example A-10 | Comparative Example A-11 |
|---|---|---|---|---|---|---|---|---|---|---|---|---|
| Total degree of acetyl substitution (DS) | | 2.4 | 2.4 | 2.4 | 2.8 | 2.4 | 1.8 | 2.4 | 2.4 | 2.4 | 2.4 | 2.4 |
| Plasticizer | Type | Triacetin | Acetyl triethyl citrate | Triacetin | Diethyl phthalate | Acetyl triethyl citrate | Diacetin | Triacetin | Triacetin | Triacetin | Triacetin | Triacetin |
| | Blending amount (part by weight) | 25 | 25 | 25 | 25 | 25 | 25 | 20 | 25 | 25 | 25 | 25 |
| First thermoplastic resin | Type | PVA | PVA | PVA | PVA | PVA | PVA | PVA | PVA | PVA | PVA | Thermoplastic starch |
| | Blending amount (part by weight) | 292 | 292 | 292 | 292 | 292 | 292 | 281 | 292 | 292 | 292 | 292 |
| Second thermoplastic resin | Type | - | - | - | - | - | - | - | - | - | - | - |
| | Blending amount (part by weight) | - | - | - | - | - | - | - | - | - | - | - |
| Cellulose acetate particles | Average particle size | 4.2 μm | 100 nm | 14 μm | 20 μm | 1.1 μm | 2.6 μm | 32 μm | 4.8 μm | 5.2 μm | 7.1 μm | 6.8 μm |
| | Coefficient of variation of particle size | 38% | 42% | 41% | 40% | 37% | 39% | 38% | 44% | 36% | 37% | 39% |
| | Sphericity | 0.98 | 0.94 | 0.97 | 0.95 | 0.97 | 0.97 | 0.96 | 0.83 | 0.98 | 0.97 | 0.95 |
| | Oil absorption (ml/100 g) | 51 | 56 | 45 | 43 | 55 | 53 | 43 | 51 | 51 | 49 | 49 |

17

| | | Compara-tive Exam-ple A-1 | Compara-tive Exam-ple A-2 | Compara-tive Exam-ple A-3 | Compara-tive Exam-ple A-4 | Compara-tive Exam-ple A-5 | Compara-tive Exam-ple A-6 | Compara-tive Exam-ple A-7 | Compara-tive Exam-ple A-8 | Compara-tive Exam-ple A-9 | Compara-tive Exam-ple A-10 | Comparative Example A-11 |
|---|---|---|---|---|---|---|---|---|---|---|---|---|
| | Degree of sur-face smooth-ness | 100% | 99% | 100% | 100% | 99% | 99% | 100% | 100% | 100% | 100% | 100% |
| | bulk specific gravity | 0.63 | 0.62 | 0.61 | 0.62 | 0.62 | 0.61 | 0.63 | 0.6 | 0.6 | 0.58 | 0.58 |
| | Theoretical specific sur-face area $(m^2/g)$ | 1.09 | 48.10 | 0.35 | 0.25 | 3.86 | 1.73 | 0.17 | 0.96 | 0.89 | 0.66 | 0.69 |
| | BET specific surface area $(m^2/g)$ | 2.84 | 91.39 | 0.84 | 0.51 | 5.79 | 2.77 | 0.35 | 2.78 | 2.40 | 0.99 | 1.24 |
| | RSSA | 2.6 | 1.9 | 2.4 | 2 | 1.5 | 1.6 | 2.1 | 2.9 | 2.7 | 1.5 | 1.8 |
| | Biodegradability | Excellent | Excellent | Excellent | Marginal | Excellent | Excellent | Excellent | Excellent | Excellent | Excellent | Excellent |
| | Tactile sensation | 3.2 | 2.7 | 3 | 2.8 | 2.6 | 3 | 2.6 | 2.7 | 3.1 | 3 | 3.1 |

[Table 3]

| | | Comparative Example A-12 | Comparative Example A-13 | Comparative Example A-14 | Comparative Example A-15 |
|---|---|---|---|---|---|
| Total degree of acetyl substitution (DS) | | 2.4 | 2.4 | 2.4 | 2.4 |
| Plasticizer | Type | Triacetin | Triacetin | Triacetin | Triacetin |
| | Blending amount (part by weight) | 25 | 25 | 25 | 25 |
| First thermoplastic resin | Type | PVA | PVA | PVA | PVA |
| | Blending amount (part by weight) | 125 | 125 | 125 | 188 |
| Second thermoplastic resin | Type | Ethylene glycol | Propylene glycol | 1,3-butylene glycol | Glycerin |
| | Blending amount (part by weight) | 25 | 25 | 25 | 21 |
| Cellulose acetate particles | Average particle size | 10.9 $\mu$m | 8.5 $\mu$m | 6.9 $\mu$m | 6.2 $\mu$m |
| | Coefficient of variation of particle size | 44% | 135% | 41% | 35% |
| | Sphericity | 0.93 | 0.84 | 0.93 | 0.95 |
| | Oil absorption (ml/100 g) | 47 | 48 | 49 | 50 |
| | Degree of surface smoothness | 99% | 99% | 100% | 100% |
| | bulk specific gravity | 0.61 | 0,61 | 0.62 | 0.62 |
| | Theoretical specific surface area (m$^2$/g) | 0.45 | 0.63 | 0.71 | 0.75 |
| | BET specific surface area (m$^2$/g) | 0.90 | 1.13 | 1.63 | 1.05 |
| | RSSA | 2 | 1.8 | 2.3 | 1.4 |
| | Biodegradability | Excellent | Excellent | Excellent | Excellent |
| | Tactile sensation | 2.9 | 3 | 3.2 | 3.1 |

As shown in Table 1-3, the cellulose acetate particles in Examples have excellent biodegradability, excellent tactile sensation, particularly soft tactile sensation, and excellent oil absorbability.

Example B-1

Preparation of liquid foundation

Components shown in Table 4 were mixed, then stirred well, and the mixture was filled into a container to prepare liquid foundation. The tactile sensation of the resulting liquid foundation was evaluated by the method described below. The results are shown in Table 12.

[Table 4]

| Component | Product name, etc. | wt.% |
|---|---|---|
| Cyclopentasiloxane | KF-995 (Shin-Etsu Chemical Co., Ltd.) | 15.2 |
| Mineral oil | HICALL K-230 (KANEDA Co., Ltd.) | 5.0 |
| Ethylhexyl methoxycinnamate | Uvinul MC80 (BASF) | 4.0 |
| Isononyl isononanoate | KAK-99 (Kokyu Alcohol Kogyo Co., Ltd.) | 3.0 |
| Disteardimonium hectorite, cyclopentasiloxane, other | Bentone Gel VS-5 PC V HV (Elementis) | 3.0 |
| Phytosteryl macadamiate | Plandool-MAS (Nippon Fine Chemical Co., Ltd.) | 0.3 |
| Trimethylsiloxysilicate, polypropylsilsesquioxane | MQ-1640 Flake Resin (Dow Coming Toray Co., Ltd.) | 0.3 |
| PEG-10 dimethicone | KF-6017P (Shin-Etsu Chemical Co., Ltd.) | 1.5 |
| Polyglyceryl oleate-2, polyhydroxystearic acid, polyglyceryl stearate-2 | PolyAquol OS2 (innovacos) | 1.0 |
| Titanium oxide, cyclopentasiloxane, other | SDL-Ti70 (Daito Kasei Kogyo Co., Ltd.) | 12.3 |
| Iron oxide, cyclopentasiloxane, other | SDL-IOY50 (Daito Kasei Kogyo Co., Ltd.) | 3.0 |
| | SDL-IOR50 (Daito Kasei Kogyo Co., Ltd.) | |
| | SDL-IOB50 (Daito Kasei Kogyo Co., Ltd.) | |
| Example A-1: cellulose acetate particles | | 3.0 |
| BG | 1,3-BG (UK) (Daicel Corporation) | 6.0 |
| Phenoxyethanol | Phenoxyethanol-SP (Yokkaichi Chemical Co., Ltd.) | 0.3 |
| Sodium chloride | | 1.0 |
| EDTA-2Na | | 0.03 |
| Purified water | | Remaining amount |
| Total | | 100.0 |

Tactile sensation

Sensory evaluation was performed according to a panel test by 20 panelists for the tactile sensation of the compositions prepared by blending the particles. Panelists were instructed to use the compositions to evaluate comprehensively both smoothness and moist feeling, on a scale with a maximum score of 5 points according to the following criteria, and an average score from 20 panelists was calculated.
Good: 5. Slightly good: 4. Average: 3. Slightly poor: 2. Poor: 1.

Example B-2

Preparation of sunscreen

Components shown in Table 5 were mixed, then stirred well, and the mixture was filled into a container to prepare a sunscreen. The tactile sensation of the resulting sunscreen was evaluated by the method described above. The results are shown in Table 12.

[Table 5]

| Component | Product name, etc. | wt.% |
|---|---|---|
| Diethylamino hydroxybenzoyl hexyl benzoate | Uvinul A Plus Glanular(BASF) | 2.00 |
| Bis-ethylhexyloxyphenol methoxyphenyl triazine | Tnosorb S(BASF) | 0.50 |
| Ethylhexyl methoxycinnamate, BHT | Uvinul MC80 (BASF) | 7.00 |
| Diisopropyl sebacate | IPSE (Nippon Fine Chemical Co., Ltd.) | 10.00 |
| Dimethicone | KF-96A-10CS (Shin-Etsu Chemical Co., Ltd.) | 2.00 |
| Isododecane | Marukasol R (Maruzen Petrochemical Co., Ltd.) | 26.47 |
| Trimethylsiloxysilicate | MQ-1640 Flake Resin (Dow Coming Toray Co., Ltd.) | 1.00 |
| PEG-9 polydimethylsiloxyethyl dimethicone | KF-6028 (Shin-Etsu Chemical Co., Ltd.) | 2.00 |
| Titanium oxide, etc. | DIS-OP-10A (Sakai Chemical Industry Co., Ltd.) | 4.00 |
| Zinc oxide, etc. | DIF-OP-3W (Sakai Chemical Industry Co., Ltd.) | 10.00 |
| Example A-1: cellulose acetate particles | | 5.00 |
| Purified water | | 19.30 |
| BG | 1,3-BG (UK) (Daicel Corporation) | 3.00 |
| Phenoxyethanol | Phenoxyethanol-SP (Yokkaichi Chemical Co., Ltd.) | 0.20 |
| Ethanol | | 7.00 |
| Sodium chloride | | 0.50 |
| EDTA-2Na | | 0.03 |
| Total | | 100.0 |

Example B-3

Preparation of powder foundation

Components A shown in Table 6 were roughly mixed, components B which had been uniformly dissolved were added thereto, and the resultant mixture was stirred well. Then, the mixture was filled into a container to prepare powder foundation. The tactile sensation of the resulting powder foundation was evaluated by the method described above. The results are shown in Table 12.

[Table 6]

| Component | wt.% |
|---|---|
| (Component (A)) | |
| Example A-1: cellulose acetate particles | 7.50 |
| SI 01-2 talc JA-46R | 29.67 |
| Mica Y-2300 | 20.00 |
| SI01-2 sericite FSE | 33.00 |
| SI 01-2 titanium oxide CR-50 | 6.50 |
| SI-2 yellow iron oxide LLXLO | 2.30 |
| SI-2 red iron oxide RED R-516L | 0.59 |
| SI-2 black iron oxide BL-100 | 0.44 |
| Component (A) total | 100.00 |

(continued)

| (Component B) | |
|---|---|
| Dimethicone (20) | 20.00 |
| Dimethicone (350) | 20.00 |
| Glyceryl isostearate | 7.20 |
| Triethylhexanoin | 17.00 |
| Octyldodecyl oleate | 31.55 |
| Sorbitan stearate | 1.00 |
| Polyglyceryl-2 oleate | 3.10 |
| Propylparaben | 0.10 |
| Tocopherol | 0.05 |
| Component (B) total | 100.0 |
| (Final blending) | |
| Component (A) | 90.00 |
| Component (B) | 100.00 |

Example B-4

Preparation of makeup base

A component C shown in Table 7 was dispersed in components A, and the mixture was stirred well. Components B were added thereto, and the resultant mixture was stirred well. Then, the mixture was filled into a container to prepare a makeup base. The tactile sensation of the resulting makeup base was evaluated by the method described above. The results are shown in Table 12.

[Table 7]

| Component | wt.% |
|---|---|
| (Component (A)) | |
| (Dimethicone/(PEG-10/5) crosspolymer, dimethicone | 3.50 |
| PEG-9 polydimethylsiloxyethyl dimethicone | 2.00 |
| Dimethicone | 5.00 |
| Isononyl isononanoate | 4.50 |
| Octyl methoxycinnamate | 10.00 |
| Quaternium-18 hectorite | 1.20 |
| (Dimethicone/vinyl dimethicone) crosspolymer, dimethicone | 5.00 |
| Cyclomethicone | 25.00 |
| (Component B) | |
| Purified water | Remainder |
| 1,3-butylene glycol | 5.00 |
| Sodium citrate | 0.20 |
| Preservative | 0.30 |
| (Component C) | |
| Example A-1: cellulose acetate particles | 10.00 |

(continued)

| | |
|---|---|
| (Component C) | |
| Total | 100.0 |

Example B-5

Preparation of lipstick base material

Components B shown in Table 8 were heated to 60°C, and mixed well. A component C was added thereto and the resultant mixture was dispersed well. Further, components A were added thereto and the resultant mixture was dissolved using a microwave oven, and then the mixture was mixed well. Then, the mixture was dissolved again by heating using the microwave oven, poured into a mold, and solidified under cooling. This solidified product was put in a lipstick container to prepare a lipstick base material. The tactile sensation of the resulting lipstick base material was evaluated by the method described above. The results are shown in Table 12.

[Table 8]

| Component | wt.% |
|---|---|
| (Component (A)) | |
| Ceresin | 4.27 |
| Microcrystalline wax | 1.55 |
| Deresinated candelilla wax | 5.03 |
| High boiling point paraffin | 3.07 |
| (Component B) | |
| Diisostearyl malate | 1.95 |
| Dipentaerythritol fatty acid ester | 6.22 |
| Adsorption refined lanolin | 2.52 |
| Liquid lanolin acetate | 13.34 |
| Glyceryl tri-2-ethylhexanoate | 19.02 |
| Liquid paraffin | 7.28 |
| Isotridecyl isononanoate | 3.21 |
| Diglyceryl triisostearate | 4.01 |
| Methylphenyl polysiloxane | 2.41 |
| Para-hydroxybenzoate | 0.07 |
| Diisostearyl malate | Remainder |
| Natural type vitamin E | 0.05 |
| (Component C) | |
| Example A-1: cellulose acetate particles | 10.00 |
| Total | 100.00 |

Example B-6

Preparation of body powder

Components A shown in Table 9 were mixed well using a mixer. The resulting body powder was filled into a container to prepare a body powder. The tactile sensation of the resulting body powder was evaluated by the method described above. The results are shown in Table 12.

[Table 9]

| Component | wt.% |
|---|---|
| (Component (A)) | |
| Talc | Remaining amount |
| Example A-1: cellulose acetate particles | 10.00 |
| Fragrance | Suitable amount |
| Total | 100.00 |

Example B-7

Preparation of solid face powder

A solid face powder was prepared in accordance with the normal method for producing a cosmetic material. That is, talc and a color pigment shown in Table 10 were mixed with a blender. Also, cellulose acetate particles and all the powder portions including the color pigment and talc mixed previously with the blender were stirred using a Henschel mixer. Thereafter, an oil component (binder) was added thereto, the mixture was heated to 70°C, further stirred, and subjected to pulverizing, as necessary. The resultant product was compression-molded into a container of a gold dish to prepare a solid face powder. The tactile sensation of the resulting solid face powder was evaluated by the method described above. The results are shown in Table 12.

[Table 10]

| Component | wt.% |
|---|---|
| (Powder) | |
| Talc | 30.00 |
| Sericite | 20.00 |
| Kaolin | 15.00 |
| Titanium oxide | 5.00 |
| Zinc myristate | 5.00 |
| Magnesium carbonate | 5.00 |
| Color pigment | Suitable amount |
| Example A-1: cellulose acetate particles | 15.00 |
| (Binder) | |
| Tragacanth gum | 3.00 |
| Liquid paraffin | 2.00 |
| Others: a preservative, an antioxidant, and a fragrance are added in a suitable amount as necessary. | |
| Total | 100.00 |

Example B-8

Preparation of solid powder eye shadow

Powders shown in Table 11 were mixed well, a binder was uniformly dissolved and added to the powders. The resultant mixture was further mixed and then compression-molded to prepare a solid powder eye shadow. The tactile sensation of the resulting solid powder eye shadow was evaluated by the method described above. The results are shown in Table 12.

[Table 11]

| Component | wt.% |
|---|---|
| (Powder) | |
| Mica | 15.00 |
| Sericite | 5.00 |
| Pigment | 15.00 |
| Pearl pigment | 10.00 |
| Example A-1: cellulose acetate particles | 51.00 |
| (Binder) | |
| Methylpolysiloxane | 2.00 |
| (Other) | |
| Sorbitan sesquioleate | 2.00 |
| Others: an antioxidant, a fragrance, and a preservative are added in a suitable amount as necessary. | |
| Total | 100.00 |

Example B-9

Liquid foundation was prepared in the same manner as in Example B-1 except that the cellulose acetate particles: Example A-1 in Table 4 were changed to the cellulose acetate particles: Example A-12. The tactile sensation of the resulting liquid foundation was evaluated by the method described above. The results are shown in Table 12.

Example B-10

A sunscreen was prepared in the same manner as in Example B-12 except that the cellulose acetate particles: Example A-1 in Table 5 were changed to the cellulose acetate particles: Example A-2. The tactile sensation of the resulting sunscreen was evaluated by the method described above. The results are shown in Table 12.

Example B-11

Powder foundation was prepared in the same manner as in Example B-12 except that the cellulose acetate particles: Example A-1 in Table 6 were changed to the cellulose acetate particles: Example A-3. The tactile sensation of the resulting powder foundation was evaluated by the method described above. The results are shown in Table 12.

Example B-12

A makeup base was prepared in the same manner as in Example B-4 except that the cellulose acetate particles: Example A-1 in Table 7 were changed to the cellulose acetate particles: Example A-12. The tactile sensation of the resulting makeup base was evaluated by the method described above. The results are shown in Table 12.

Example B-13

Liquid foundation was prepared in the same manner as in Example B-1 except that the cellulose acetate particles: Example A-1 in Table 4 were changed to the cellulose acetate particles: Example A-12. The tactile sensation of the resulting liquid foundation was evaluated by the method described above. The results are shown in Table 12.

Example B-14

A sunscreen was prepared in the same manner as in Example B-12 except that the cellulose acetate particles: Example A-1 in Table 5 were changed to the cellulose acetate particles: Example A-2. The tactile sensation of the resulting sunscreen was evaluated by the method described above. The results are shown in Table 12.

Example B-15

Liquid foundation was prepared in the same manner as in Example B-1 except that the cellulose acetate particles: Example A-1 in Table 4 were changed to the cellulose acetate particles: Example A-12. The tactile sensation of the resulting liquid foundation was evaluated by the method described above. The results are shown in Table 12.

Example B-16

A sunscreen was prepared in the same manner as in Example B-12 except that the cellulose acetate particles: Example A-1 in Table 5 were changed to the cellulose acetate particles: Example A-2. The tactile sensation of the resulting sunscreen was evaluated by the method described above. The results are shown in Table 12.

Example B-17

Liquid foundation was prepared in the same manner as in Example B-1 except that cyclopentasiloxane in Table 4 was replaced with a mixture prepared by mixing dodecane (PARAFOL 12-97 (Sasol)) and Cetiol Ultimate (undecane: tridecane = 65 wt.%: 35 wt.%, available from BASF) in an identical weight ratio, isononyl isononanoate was replaced with a mixture prepared by mixing coco-caprylate (Cetiol C5 (BASF)), coco-caprylate/caprate (Cetiol C5C (BASF)), and dicaprylyl carbonate (Cetiol CC (BASF)) in an identical weight ratio, and phytosteryl macadamiate was replaced with camellia oil (Pure Tsubaki oil (Nikko Rica Corporation)). The tactile sensation of the resulting liquid foundation was evaluated by the method described above. The results are shown in Table 12.

Example B-18

A sunscreen was prepared in the same manner as in Example B-2 except that isododecane in Table 5 was replaced with a mixture prepared by mixing dodecane (PARAFOL 12-97 (Sasol)) and Cetiol Ultimate (undecane: tridecane = 65 wt.%: 35 wt.%, available from BASF) in an identical weight ratio, and diisopropyl sebacate was replaced with a mixture prepared by mixing coco-caprylate (Cetiol C5 (BASF)), coco-caprylate/caprate (Cetiol C5C (BASF)), and dicaprylyl carbonate (Cetiol CC (BASF)) in an identical weight ratio. The tactile sensation of the resulting sunscreen was evaluated by the method described above. The results are shown in Table 12.

Example B-19

Powder foundation was prepared in the same manner as in Example B-3 except that dimethicone in Table 6 was replaced with a mixture prepared by mixing dodecane (PARAFOL 12-97 (Sasol)) and Cetiol Ultimate (undecane: tridecane = 65 wt.%: 35 wt.%, available from BASF) in an identical weight ratio, and octyldodecyl oleate was replaced with a mixture prepared by mixing coco-caprylate (Cetiol C5 (BASF)), coco-caprylate/caprate (Cetiol C5C (BASF)), and dicaprylyl carbonate (Cetiol CC (BASF)) in an identical weight ratio. The tactile sensation of the resulting powder foundation was evaluated by the method described above. The results are shown in Table 12.

Example B-20

A makeup base was prepared in the same manner as in Example B-4 except that cyclomethicone in Table 7 was replaced with a mixture prepared by mixing dodecane (PARAFOL 12-97 (Sasol)) and Cetiol Ultimate (undecane: tridecane = 65 wt.%: 35 wt.%, available from BASF) in an identical weight ratio, and isononyl isononanoate was replaced with a mixture prepared by mixing coco-caprylate (Cetiol C5 (BASF)), coco-caprylate/caprate (Cetiol C5C (BASF)), and dicaprylyl carbonate (Cetiol CC (BASF)) in an identical weight ratio. The tactile sensation of the resulting makeup base was evaluated by the method described above. The results are shown in Table 12.

Example B-21

Powder foundation was prepared in the same manner as in Example B-3 except that Mica Y-2300X in Table 6 was replaced with a mixture prepared by mixing mica (Mica Y-2300X (Yamaguchi Mica Co., Ltd.)), synthetic mica (PDM-10L (Topy Industries Limited), and (fluorinated/hydroxylated/oxidized)/(Mg/K/silicon) (Micro Mica MK-200K (Katakura & Co-op Agri Corporation) in an identical weight ratio, and sericite was replaced with a mixture prepared by mixing barium sulfate (plate-like barium sulfate H, available from Sakai Chemical Industry Co., ltd.) and boron nitride (SHP-6 (Mizushima Ferroalloy Co., Ltd.)) in an identical weight ratio, and talc was replaced with a mixture prepared by mixing cellulose (NP fiber W-06MG (Nippon Paper Industries Co., Ltd.)) and silica (Godd Ball E-16C (Suzukiyushi Industrial Corporation)) in an identical weight ratio. The tactile sensation of the resulting powder foundation was evaluated by the method described above. The results are shown in Table 12.

Example B-22

A body powder was prepared in the same manner as in Example B-6 except that talc in Table 9 was replaced with a mixture prepared by mixing cellulose (NP fiber W-06MG (Nippon Paper Industries Co., Ltd.)) and silica (Godd Ball E-16C (Suzukiyushi Industrial Corporation)) in an identical weight ratio. The tactile sensation of the resulting body powder was evaluated by the method described above. The results are shown in Table 12.

Example B-23

A solid powder eye shadow was prepared in the same manner as in Example B-8 except that Mica Y-2300X in Table 11 was replaced with a mixture prepared by mixing mica (Mica Y-2300X (Yamaguchi Mica Co., Ltd.)), synthetic mica (PDM-10L (Topy Industries Limited)), and (fluorinated/hydroxylated/oxidized)/(Mg/K/silicon) (Micro Mica MK-200K (Katakura & Co-op Agri Corporation) in an identical weight ratio, and sericite was replaced with a mixture prepared by mixing barium sulfate (plate-like barium sulfate H (Sakai Chemical Industry Co., Ltd.)) and boron nitride (SHP-6 (Mizushima Ferroalloy Co., Ltd.)) in an identical weight ratio. The tactile sensation of the resulting solid powder eye shadow was evaluated by the method described above. The results are shown in Table 12.

Example B-24

A liquid foundation was prepared in the same manner as in Example B-1 except that BG in Table 4 was replaced with a mixture prepared by mixing glycerin and pentylene glycol (Diol PD (Kokyu Alcohol Kogyo Co., Ltd.)) in an identical weight ratio. The tactile sensation of the resulting liquid foundation was evaluated by the method described above. The results are shown in Table 12.

Example B-25

A sunscreen was prepared in the same manner as in Example B-2 except that BG in Table 5 was replaced with a mixture prepared by mixing glycerin and pentylene glycol (Diol PD (Kokyu Alcohol Kogyo Co., Ltd.)) in an identical weight ratio. The tactile sensation of the resulting sunscreen was evaluated by the method described above. The results are shown in Table 12.

Example B-26

A makeup base was prepared in the same manner as in Example B-4 except that 1,3-butylene glycol in Table 7 was replaced with a mixture prepared by mixing glycerin and pentylene glycol (Diol PD (Kokyu Alcohol Kogyo Co., Ltd.)) in an identical weight ratio. The tactile sensation of the resulting makeup base was evaluated by the method described above. The results are shown in Table 12.

Comparative Examples B-1 to B-8

In Comparative Examples B-1 to B-8, liquid foundation, a sunscreen, powder foundation, a makeup base, a lipstick base, a body powder, a solid face powder, and a solid powder eye shadow were prepared in the same manner as in Examples B-1 to B-8 except that cellulose acetate particles in Example A-1 in Tables 4 to 11 were replaced with cellulose acetate particles in Comparative Example A-1. The tactile sensation of each of the products was evaluated by the method described above. The results are shown in Table 13.

[Table 12]

| | Example B-1 | Example B-2 | Example B-3 | Example B-4 | Example B-5 | Example B-6 | Example B-7 | Example B-8 |
|---|---|---|---|---|---|---|---|---|
| Composition | Liquid foundation | Sunscreen | Powder foundation | Makeup base | Lipstick base | Body powder | Solid face powder | Solid powder eye shadow |
| Particles | Example A-1 | Example A-1 | Example A-1 | Example A-1 | Example A-1 | Example A-1 | Example A-1 | Example A-1 |
| Tactile sensation | 4.5 | 4.6 | 4.3 | 4.2 | 4.1 | 4.5 | 4.7 | 4.6 |
| | Example B-9 | Example B-10 | Example B-11 | Example B-12 | Example B-13 | Example B-14 | Example B-15 | Example B-16 |
| Composition | Liquid foundation | Sunscreen | Powder foundation | Makeup base | Liquid foundation | Sunscreen | Liquid foundation | Sunscreen |
| Particles | Example A-12 | Example A-12 | Example A-12 | Example A-12 | Example A-12 | Example A-12 | Example A-12 | Example A-12 |
| Tactile sensation | 4.1 | 4.2 | 4.1 | 4.3 | 4.3 | 4.4 | 4.1 | 4.1 |
| | Example B-17 | Example B-18 | Example B-19 | Example B-20 | Example B-21 | Example B-22 | Example B-23 | Example B-24 |
| Composition | Liquid foundation | Sunscreen | Powder foundation | Makeup base | Powder foundation | Body powder | Solid powder eye shadow | Liquid foundation |
| Particles | Example A-1 | Example A-1 | Example A-1 | Example A-1 | Example A-1 | Example A-1 | Example A-1 | Example A-1 |
| Tactile sensation | 4.2 | 4.3 | 4.2 | 4.1 | 4.2 | 4.4 | 4.2 | 4.3 |

(continued)

| Composition | Liquid foundation | Sunscreen | Powder foundation | Makeup base | Powder foundation | Body powder | Solid powder eye shadow | Liquid foundation |
|---|---|---|---|---|---|---|---|---|
| | Example B-25 | Example B-26 | | | | | | |
| Composition | Sunscreen | Makeup base | | | | | | |
| Particles | Example A-1 | Example A-1 | | | | | | |
| Tactile sensation | 4.3 | 4.3 | | | | | | |

[Table 13]

| | Comparative Example B-1 | Comparative Example B-2 | Comparative Example B-3 | Comparative Example B-4 | Comparative Example B-5 | Comparative Example B-6 | Comparative Example B-7 | Comparative Example B-8 |
|---|---|---|---|---|---|---|---|---|
| Composition | Liquid foundation | Sunscreen | Powder foundation | Makeup base | Lipstick base | Body powder | Solid face powder | Solid powder eye shadow |
| Particles | Comparative Example A-1 | Comparative Example A-1 | Comparative Example A-1 | Comparative Example A-1 | Comparative Example A-1 | Comparative Example A-1 | Comparative Example A-1 | Comparative Example A-1 |
| Tactile sensation | 3.1 | 2.9 | 3.2 | 3.1 | 3.3 | 3.2 | 3.2 | 3.1 |

As shown in Tables 12 and 13, all of the cosmetic compositions containing cellulose acetate particles of Examples B-1 to B-26 have a tactile sensation score of 4.0 or greater, particularly soft tactile sensation, and are excellent. Further, the cosmetic compositions contain cellulose acetate particles, and thus have excellent biodegradability.

**Claims**

1.  Cellulose acetate particles having:

    an average particle size of 80 nm or greater and 100 $\mu$m or less, a sphericity of 0.7 or greater and 1.0 or less, and a relative specific surface area of 3.0 or greater and 20 or less; and
    a total degree of acetyl substitution of the cellulose acetate of 0.7 or greater and 3.0 or less.

2.  The cellulose acetate particles according to claim 1, having a degree of surface smoothness of 10% or greater and 95% or less.

3.  The cellulose acetate particles according to claim 1 or 2, having a bulk specific gravity of 0.2 or greater and 0.7 or less.

4.  The cellulose acetate particles according to any one of claims 1 to 3, having an oil absorption using linseed oil of 60 ml or greater per 100 g of the cellulose acetate particles.

5.  The cellulose acetate particles according to any one of claims 1 to 4, having the total degree of acetyl substitution of the cellulose acetate of 1.6 or greater and less than 2.9.

6.  The cellulose acetate particles according to any one of claims 1 to 5, having the relative specific surface area of 10 or greater and 20 or less.

7.  The cellulose acetate particles according to any one of claims 1 to 6, wherein the cellulose acetate particles contain a plasticizer, and a content of the plasticizer is 2 parts by weight or greater and 67 parts by weight or less relative to 100 parts by weight of the cellulose acetate.

8.  The cellulose acetate particles according to claim 7, wherein the plasticizer contains at least one selected from the group consisting of a citrate-based plasticizer, a glycerin ester-based plasticizer, and a phthalate-based plasticizer.

9.  A cosmetic composition comprising the cellulose acetate particles described in any one of claims 1 to 8.

10. A method for producing cellulose acetate particles, comprising:

    mixing cellulose acetate having a total degree of acetyl substitution of 0.7 or greater and 3.0 or less, a plasticizer, a first thermoplastic polymer, and a second thermoplastic polymer, and forming a mixture of cellulose acetate containing the plasticizer, the first thermoplastic polymer, and the second thermoplastic polymer;
    melt-kneading the mixture at 200°C or higher and 280°C or lower; and
    removing the first thermoplastic polymer and the second thermoplastic polymer from the melt-kneaded mixture, wherein, when SPa represents an SP value of the cellulose acetate, SPb represents an SP value of the first thermoplastic polymer, and SPc represents an SP value of the second thermoplastic polymer, SPa, SPb, and SPc satisfy the following relation:

    $$0.1 \leq |\,SPc - SPa\,| \,/\, |\,SPb - SPa\,| \leq 0.9.$$

11. The method for producing cellulose acetate particles according to claim 10, wherein the plasticizer contains at least one selected from the group consisting of acetyl triethyl citrate and triacetin.

12. The method for producing cellulose acetate particles according to claim 10 or 11, wherein the first thermoplastic polymer contains at least one selected from the group consisting of polyvinyl alcohol and thermoplastic starch.

13. The method for producing cellulose acetate particles according to any one of claims 10 to 12, wherein the second thermoplastic polymer is polyethylene glycol.

HL          x3.0k      30 um

FIG. 1

HL      x5.0k   20 um

# FIG. 2

FIG. 3

SU5000 1.0kV 8.0mm x6.00k SE(L)                    5.00μm

# FIG. 4

HL    x3.0k    30 um

# FIG. 5

HL          x5.0k      20 um

FIG. 6

HL    x3.0k    30 um

FIG. 7

HL          x5.0k     20 um

FIG. 8

## INTERNATIONAL SEARCH REPORT

International application No.

PCT/JP2021/025809

### A. CLASSIFICATION OF SUBJECT MATTER

A61Q 1/02(2006.01)i; A61Q 1/04(2006.01)i; A61Q 1/10(2006.01)i; A61Q 17/04(2006.01)i; A61Q 19/00(2006.01)i; A61K 8/37(2006.01)i; A61K 8/73(2006.01)i

FI:  A61K8/73; A61K8/37; A61Q1/02; A61Q1/04; A61Q1/10; A61Q17/04; A61Q19/00

According to International Patent Classification (IPC) or to both national classification and IPC

### B. FIELDS SEARCHED

Minimum documentation searched (classification system followed by classification symbols)

A61K8/00-99, A61Q1/00-90/00, C08B3/06-10, C08L1/12, C08J3/12

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2019/156116 A1 (DAICEL CORPORATION) 15 August 2019 (2019-08-15) paragraphs [0067]-[0148], fig. 1 | 1-13 |
| A | JP 11-255959 A (DICEL CHEMICAL INDUSTRIES, LTD.) 21 September 1999 (1999-09-21) paragraph [0014] | 1-13 |

☐ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 August 2021 (11.08.2021) | 31 August 2021 (31.08.2021) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

INTERNATIONAL SEARCH REPORT
Information on patent family members

International application No.

PCT/JP2021/025809

| Patent Documents referred in the Report | Publication Date | Patent Family | Publication Date |
|---|---|---|---|
| WO 2019/156116 A1 | 15 Aug. 2019 | US 2020/0179261 A1 paragraphs [0071]-[0149], fig. 1<br>EP 3613794 A1<br>CN 110650994 A<br>KR 10-2019-0135537 A | |
| JP 11-255959 A | 21 Sep. 1999 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S62215638 A
- JP 6609726 B
- JP 2015214690 A
- JP H06136175 A
- JP 4464815 B
- JP H06006307 B
- WO 9204408 A

**Non-patent literature cited in the description**

- **TEZUKA.** *Carbonydr. Res.,* 1995, vol. 273, 83